(19) Europäisches Patentamt
European Patent Office
Office européen des brevets

(11) Veröffentlichungsnummer: **0 084 600**
**B1**

# (12) EUROPÄISCHE PATENTSCHRIFT

(45) Veröffentlichungstag der Patentschrift: **25.06.86**

(51) Int. Cl.⁴: **A 01 N 37/34**, A 01 N 43/20, A 01 N 33/18, A 01 N 29/08, C 07 C 121/48, C 07 D 303/48

(21) Anmeldenummer: **82109135.2**

(22) Anmeldetag: **04.10.82**

(54) **Substituierte bicyclische Verbindungen und ihre Anwendung als Schädlingsbekämpfungsmittel.**

(30) Priorität: **16.10.81 DE 3141119**

(43) Veröffentlichungstag der Anmeldung: **03.08.83 Patentblatt 83/31**

(45) Bekanntmachung des Hinweises auf die Patenterteilung: **25.06.86 Patentblatt 86/26**

(84) Benannte Vertragsstaaten: **BE CH DE FR GB IT LI NL**

(56) Entgegenhaltungen:
**FR - A - 2 077 226**
**US - A - 2 536 858**
**US - A - 2 635 979**
**US - A - 2 793 975**
**US - A - 3 335 166**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Kölbl, Heinz, Dr., Andreas-Gryphius-Strasse 20, D-5000 Köln 80 (DE)**
Erfinder: **Gompper, Rudolf, Prof. Dr., Thaddäus-Eck-Strasse 34, D-8000 München 60 (DE)**
Erfinder: **Behrenz, Wolfgang, Dr., Untergründemich 14, D-5063 Overath (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**
Erfinder: **Hermann, Günther, Dr., Paul-Klee-Strasse 64, D-5090 Leverkusen 1 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von bicyclischen Verbindungen als Schädlingsbekämpfungsmittel.

Es ist bereits bekannt geworden, dass hochchlorierte bicyclische Verbindungen insektizide Wirksamkeit besitzen (US-P 2 546 174).

Es ist ferner bekannt geworden, dass 5-Nitro-6-trichlor-methyl-norbornen insektizide Wirksamkeit besitzt (Chemical Abstracts Bnd. 60 Zitat 5360e).

Die Wirkung dieser Verbindungen ist jedoch, vor allem bei niedrigen Aufwandkonzentrationen, nicht immer voll befriedigend.

Die Erfindung betrifft die Verwendung zur Bekämpfung von Schädlingen von einem substituierten Bicyclus der allgemeinen Formel I

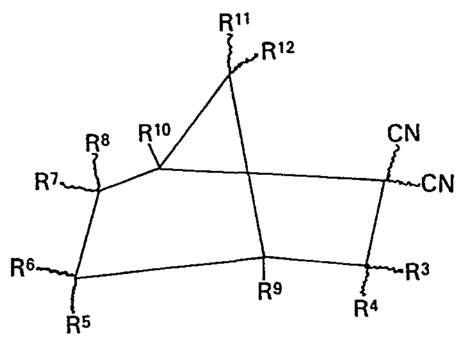
I

in welcher

$R^3$ für Wasserstoff, $C_1$–$C_4$-Alkyl, gegebenenfalls durch Halogen, Alkyl oder $NO_2$ substituiertes Phenyl, Halogen, $C_{1-4}$-Halogenalkyl, Acetoxy, $C_1$–$C_4$-Alkoxycarbonyl, Aralkoxycarbonyl, Phenyloxycarbonyl, Cyano, Nitro, Hydroxy, $C_1$–$C_4$-Alkoxy, Phenyloxy,

$R^4$ für Wasserstoff, $C_1$–$C_4$-Alkyl, gegebenenfalls durch Halogen oder $NO_2$ substituiertes Phenyl, Halogen, $C_{1-4}$-Halogenalkyl, $C_{1-4}$-Alkoxycarbonyl oder $R^3$ und $R^4$ zusammen für Dihalogenmethylen,

$R^5$, $R^6$, $R^7$, $R^8$ unabhängig voneinander für Wasserstoff, $C_1$–$C_4$-Alkyl, Phenyl, Halogen, $C_{1-4}$-Halogenalkyl, $C_1$–$C_4$-Alkylthio, Phenylthio, Phenoxy, $C_1$–$C_4$-Alkoxy, und darüber hinaus $R^5$ und $R^6$ zusammen für eine Doppelbindung oder Sauerstoff oder

$R^6$ und $R^7$ zusammen für eine Doppelbindung oder für Sauerstoff stehen,

$R^9$ und $R^{10}$ unabhängig voneinander für Wasserstoff, $C_1$–$C_4$-Alkyl, $C_{1-4}$-Halogenalkyl, Fluor,

$R^{11}$ und $R^{12}$ unabhängig voneinander für Wasserstoff, $C_{1-4}$-Alkyl, $C_{1-4}$-Halogenalkyl, Halogen oder $R^{11}$ und $R^{12}$ zusammen für Ethyliden, Isopropyliden stehen.

Insbesonders bevorzugt sind Schädlingsbekämpfungsmittel der Formel I, in welcher

$R^3$ für Wasserstoff, Methyl, Ethyl, Brom, Chlor, Fluor, Cyano, Trifluormethyl, Trichlormethyl, Acetoxy, Hydroxy, Dichlorfluormethyl, Chlordifluormethyl, Bromdichlormethyl, Phenyl, o-Chlorphenyl, p-Nitrophenyl, Methoxycarbonyl, Ethoxycarbonyl, Pentafluorbenzyloxycarbonyl, Phenoxycarbonyl steht,

$R^4$ für Wasserstoff, Methyl, Ethyl, Brom, Chlor, Fluor, Trifluormethyl, Trichlormethyl, Dichlorfluormethyl, Chlordifluormethyl, Bromdichlormethyl, Phenyl, o-Chlorphenyl, p-Nitrophenyl, Methocycarbonyl, Ethoxycarbonyl steht,

$R^3$ und $R^4$ zusammen für Dichlormethylen stehen,

$R^5$, $R^6$, $R^7$, $R^8$ unabhängig voneinander für Wasserstoff, Methyl, Brom, Chlor, Fluor, Methylthio, Phenylthio stehen,

$R^6$ und $R^7$ zusammen für eine Doppelbindung oder Sauerstoff stehen,

$R^9$ und $R^{10}$ unabhängig voneinander für Wasserstoff, Methyl, Trifluormethyl, Fluor stehen,

$R^{11}$ und $R^{12}$ unabhängig voneinander für Wasserstoff, Methyl, Fluor, Brom, Chlor, 1-Bromethyl, 1-Chlorethyl, 1-Brom-1-Methylethyl, 1-Chlor-1-Methylethyl stehen,

$R^{11}$ und $R^{12}$ zusammen für Ethyliden, Isopropyliden stehen.

Die Erfindung betrifft ebenfalls neue Schädlingsbekämpfungsmittel gekennzeichnet durch einen Gehalt an mindestens einem substituierten Bicyclus der obigen Formel I mit der Ausnahme von 3,3-bis-Trifluormethyl-bicyclo[2.2.1]hept-5-en-2,2-dicarbonitril.

Überraschenderweise zeigen die substituierten Bicyclen der Formel I eine bessere insektizide Wirkung als die aus dem Stand der Technik bekannten Verbindungen. Sie wirken ausserdem als Rodentizide. Die erfindungsgemässen Wirkstoffe werden durch die Formel (I) definiert.

Aus US-A-3 335 166, US-A-2 536 858 sind strukturell verwandte Mononitrile bekannt, die den erfindungsgemässen Dinitrilen jedoch wirkungsmässig unterlegen sind.

Aus dem Artikel von W.J. Middleton in The Journal of Organic Chemistry Vol. 30, 1402 ff, sind polycyclische Dinitrile bekannt, die sich von den erfindungsgemässen Verbindungen jedoch strukturell wesentlich unterscheiden. Weiterhin ist von einer Verwendung dieser Strukturen als Schädlingsbekämpfungsmittel nichts bekannt geworden.

Im einzelnen seien folgende Verbindungen benannt:

3,3-Dichlor-bicyclo[2.2.1]hept-5-en2,2-dicarbonitril
3,3-Dichlor-bicyclo[2.2.1]heptan2,2-dicarbonitril
3-Chlor-bicyclo[2.2.1]hept-5-en-2,2-dicarbonitril
3-Chlor-bicyclo[2.2.1]heptan-2,2-dicarbonitril
5,6-Dibrom-3,3-dichlor-bicyclo[2.2.1]heptan-2,2-dicarbonitril
3,3,5,6-Tetrachlor-bicyclo[2.2.1]heptan-2,2-dicarbonitril
5-Brom-3,3-dichlor-bicyclo[2.2.1]hept-5-en-2,2-dicarbonitril
6-Brom-3,3-dichlor-bicyclo[2.2.1]hept-5-en-2,2-dicarbonitril
3,3-Dichlor-5,6-epoxy-bicyclo[2.2.1]heptan-2,2-dicarbonitril

3,3-bis-(Trifluormethyl)-bicyclo[2.2.1]hept-5-en-2,2-dicarbonitril
3,3-bis-(Trifluormethyl)-bicyclo[2.2.1]heptan-2,2-dicarbonitril
3-Chlor-3-trifluormethyl-bicyclo[2.2.1]hept-5-en-2,2-dicarbonitril
Bicyclo[2.2.1]hept-5-en-2,2-dicarbonitril
3,3-Dichlor-5,6-epoxy-bicyclo[2.2.1]heptan-2,2-dicarbonitril
3-Acetoxy-bicyclo[2.2.1]hept-5-en-2,2-dicarbonitril
5,6-Dibrom-3,3-bis(trifluormethyl)-bicyclo[2.2.1]-heptan-2,2-dicarbonitril
3-Chlor-methyl-bicyclo[2.2.1]hept-5-en-2,2-dicarbonitril
Bicyclo[2.2.1]heptan-2,2-dicarbonitril
2,3-Dichlor-bicyclo[2.2.1]hept-5-en-2,3-dicarbonitril
5,6-Dibrom-3-chlor-bicyclo[2.2.1]heptan-2,2-dicarbonitril

Die Verbindungen der allgemeinen Formel (Iaa) sind neu:

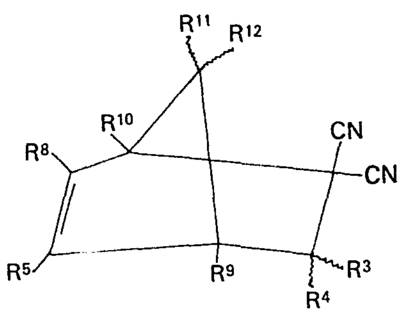

Iaa

wobei
$R^3$ für Chlor, Brom, Fluor, $C_1$–$C_4$-Alkyl, Trifluormethyl, Acetoxy steht,
$R^4$ für Wasserstoff, Brom, Fluor, Methyl, gegebenenfalls durch Halogen oder $NO_2$ substituiertes Phenyl steht,
$R^5$, $R^8$, $R^9$, $R^{10}$, $R^{11}$, $R^{12}$ unabhängig voneinander für Wasserstoff, Methyl, Ethyl, Halogenmethyl, Trifluormethyl, Fluor stehen.

Verbindungen der Formel (Iaa) werden erhalten, indem man ein Cyclopentadien der allgemeinen Formel (II),

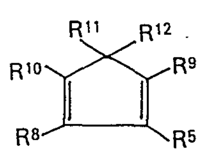

II

wobei $R^5$, $R^8$, $R^9$, $R^{10}$, $R^{11}$, $R^{12}$, die oben angegebene Bedeutung haben, mit einem Alken der allgemeinen Formel (III).

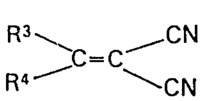

III

wobei $R^3$ und $R^4$ die oben angegebene Bedeutung haben, umsetzt.

Dabei wird das Olefin der Formel (III) gegebenenfalls in einem Lösungsmittel mit dem Cyclopentadien der Formel (II) vermischt bzw. beide Komponenten gegebenenfalls in einem Lösungsmittel zusammengetropft.

Als Lösungsmittel kommen in Frage: Alkane, wie z.B. Pentan, Hexan, Petrolether, Ligroin; Cycloalkane, wie z.B. Cyclohexan; Aromaten, wie z.B. Benzol, Toluol, Xylol, Chlorbenzol; cyclische Ether, wie z.B. Tetrahydrofuran, Dioxan; Halogenkohlenwasserstoffe, wie z.B. Dichlormethan, Trichlormethan, Tetrachlormethan.

Die Umsetzung kann bei Temperaturen von −20°C bis 250°C erfolgen, bevorzugt bei 20–100°C.

Das Cyclopentadien II wird bevorzugt als Monomeres eingesetzt in einem molaren Verhältnis von 1:1 bis 1:10, bevorzugt 1:1 bis 1:3, bezogen auf das Olefin (III). Bei einer Reaktionstemperatur von über 120°C, bevorzugt ab 150°C, kann auch ein Dimeres des Cyclopentadiens (II) eingesetzt werden.

Die neuen Verbindungen der allgemeinen Formel (Ib)

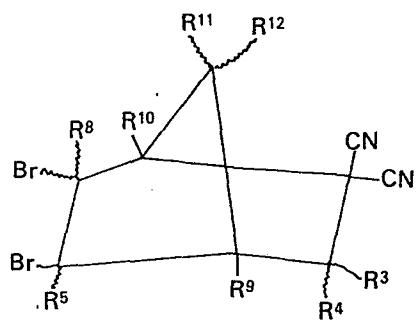

Ib

in welcher
$R^3$ für Chlor, Brom, Fluor, $C_1$–$C_4$-Alkyl, Trifluormethyl, Acetoxy, gegebenenfalls durch Halogen, Alkyl oder $NO_2$ substituiertes Phenyl, Cyano steht,
$R^4$ für Wasserstoff, Chlor, Brom, Fluor, $C_1$–$C_4$-Alkyl, Trifluormethyl, gegebenenfalls durch Halogen oder $NO_2$ substituiertes Phenyl steht und
$R^5$, $R^8$, $R^9$, $R^{10}$, $R^{11}$, $R^{12}$ unabhängig voneinander für Wasserstoff, Methyl, Ethyl, Halogenmethyl, Trifluormethyl, Fluor stehen,
werden hergestellt, indem man eine Verbindung der allgemeinen Formel Ia

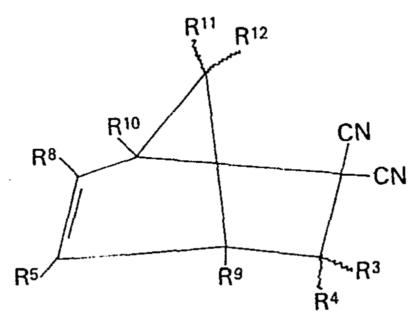

Ia

in welcher
$R^3$ für Chlor, Brom, Fluor, $C_1$–$C_4$-Alkyl, Trifluormethyl, Acetoxy, gegebenenfalls durch Halogen,

Alkyl oder $NO_2$ substituiertes Phenyl, Cyano steht,
$R^4$ für Wasserstoff, Chlor, Brom, Fluor, $C_1$-$C_4$-Alkyl, Trifluormethyl, gegebenenfalls durch Halogen oder $NO_2$ substituiertes Phenyl steht und
$R^5$, $R^8$, $R^9$, $R^{10}$, $R^{11}$, $R^{12}$ die oben angegebenen Bedeutung haben, mit Brom gegebenenfalls in einem Lösungsmittel umsetzt.

Das Brom wird in einem molaren Verhältnis von 1:1 bis 1:10, vorzugsweise 1:1 bis 1:3, bezogen auf (Ia), gegebenenfalls in demselben Lösungsmittel zugetropft. Als Lösungsmittel kommen in Frage: chlorierte Kohlenwasserstoffe, wie z.B. Dichlormethan, Trichlormethan, Tetrachlormethan; Alkane, wie z.B. Pentan, Hexan; Essigsäure.

Die Reaktion wird bei einer Temperatur von 0°C bis 100°C, vorzugsweise 20°C bis 80°C, durchgeführt. Nach erfolgter Umsetzung werden das Lösungsmittel und gegebenenfalls überschüssiges Brom abdestilliert und der Rückstand destilliert oder umkristallisiert.

Die neuen Verbindung der allgemeinen Formel (Ic)

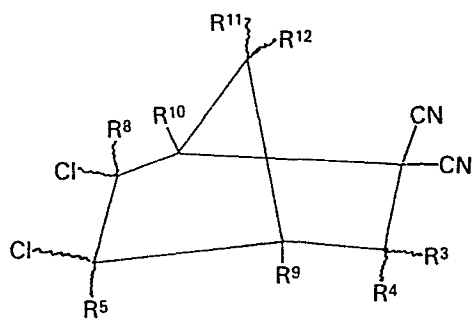

Ic

in welcher
$R^3$ für Chlor, Brom, Fluor, $C_1$-$C_4$-Alkyl, Trifluormethyl, Acetoxy, gegebenenfalls durch Halogen, Alkyl oder $NO_2$ substituiertes Phenyl, Cyano steht,
$R^4$ für Wasserstoff, Chlor, Brom, Fluor, $C_1$-$C_4$-Alkyl, Trifluormethyl, gegebenenfalls durch Halogen oder $NO_2$ substituiertes Phenyl steht und
$R^5$, $R^8$, $R^9$, $R^{10}$, $R^{11}$, $R^{12}$ unabhängig voneinander für Wasserstoff, Methyl, Ethyl, Halogenmethyl, Trifluormethyl, Fluor stehen,
werden hergestellt, indem man eine Verbindung der Formel (Ia)

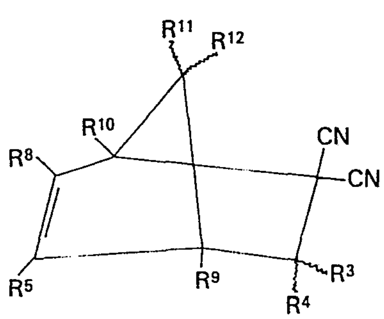

Ia

in welcher
$R^3$ für Chlor, Brom, Fluor, $C_1$-$C_4$-Alkyl, Trifluormethyl, Acetoxy, gegebenenfalls durch Halogen, Alkyl oder $NO_2$ substituiertes Phenyl, Cyano steht,
$R^4$ für Wasserstoff, Chlor, Brom, Fluor, $C_1$-$C_4$-Alkyl, Trifluormethyl, gegebenenfalls durch Halogen oder $NO_2$ substituiertes Phenyl steht und
$R^5$, $R^8$, $R^9$, $R^{10}$, $R^{11}$, $R^{12}$ die oben angegebene Bedeutung haben,
mit Chlor umsetzt.

Die Umsetzung erfolgt bevorzugt in einem chlorierten Kohlenwasserstoff, wie z.B. Dichlormethan, Trichlormethan, Tetrachlormethan, und bei einer Temperatur von 0°C bis 100°C, vorzugsweise 20–80°C. Dabei wird ein Chlorstrom durch die Mischung geleitet, bis die Verbindung der Formel Ia vollständig umgesetzt ist. Gegebenenfalls kann die Reaktion durch Belichtung mit einer UV- oder Tageslichtlampe unterstützt werden. Das Lösungsmittel wird abdestilliert und der Rückstand destilliert oder umkristallisiert.

Die neuen Verbindungen der Formel (Id),

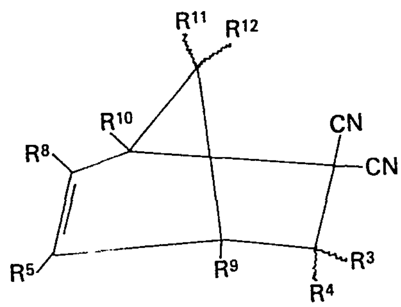

Id

in welcher
$R^3$ für Chlor, Brom, Fluor, $C_1$-$C_4$-Alkyl, Trifluormethyl, Acetoxy, gegebenenfalls durch Halogen, Alkyl oder $NO_2$ substituiertes Phenyl, Cyano steht,
$R^4$ für Wasserstoff, Chlor, Brom, Fluor, $C_1$-$C_4$-Alkyl, Trifluormethyl, gegebenenfalls durch Halogen oder $NO_2$ substituiertes Phenyl steht und
$R^5$, $R^8$, $R^9$, $R^{10}$, $R^{11}$, $R^{12}$ unabhängig voneinander für Wasserstoff, Methyl, Ethyl, Halogenmethyl, Trifluormethyl, Fluor stehen,
wobei einer der Reste $R^5$ und $R^8$ für Chlor steht und der andere die angegebene Bedeutung hat,
werden hergestellt, indem man eine Verbindung der Formel (Ic), wobei mindestens einer der Reste $R^5$ und $R^8$ für Wasserstoff stehen muss, gegebenenfalls in einem Lösungsmittel mit einer Base dehydrochloriert.

Als Lösungsmittel kommen in Frage chlorierte Kohlenwasserstoffe, wie z.B. Dichlormethan, Trichlormethan, Tetrachlormethan; Aromaten, wie z.B. Benzol, Toluol, Xylol, Chlorbenzol; Alkane, wie z.B. Pentan, Hexan; cyclische Ether, wie z.B. Tetrahydrofuran, Dioxan.

Als Basen kommen in Frage Amine, bevorzugt teritäre Amine, wie z.B. Triethylamin, Diazabicycloundecen, Diazabicyclononen, Diisopropylethylamin, Pyridin, Chinolin.

Die Reaktion wird bei einer Temperatur von 0°C bis 120°C, bevorzugt bei 20°C–80°C durchgeführt.

Die neuen Verbindungen der Formel (Ie)

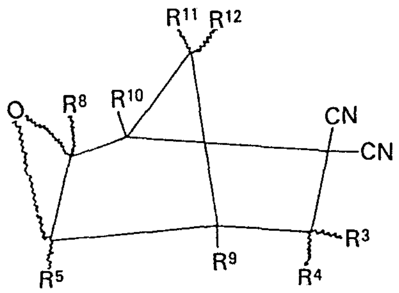

Ie

in welcher
$R^3$ für Chlor, Brom, Fluor, $C_1$–$C_4$-Alkyl, Trifluormethyl, Acetoxy, gegebenenfalls durch Halogen, Alkyl oder $NO_2$ substituiertes Phenyl, Cyano steht,
$R^4$ für Wasserstoff, Chlor, Brom, Fluor, $C_1$–$C_4$-Alkyl, Trifluormethyl, gegebenenfalls durch Halogen oder $NO_2$ substituiertes Phenyl steht und
$R^5$, $R^8$, $R^9$, $R^{10}$, $R^{11}$, $R^{12}$ unabhängig voneinander für Wasserstoff, Methyl, Ethyl, Halogenmethyl, Trifluormethyl, Fluor stehen,
werden hergestellt, indem man eine Verbindung der Formel (Ia) mit einer Persäure reagieren lässt.

Die Umsetzung erfolgt bevorzugt in einem chlorierten Kohlenwasserstoff, wie z.B. Dichlormethan, Trichlormethan oder in einem Aromaten, wie z.B. Benzol, Toluol, Chlorbenzol. Dazu wird im Verhältnis 1:1 bis 1:2 eine Lösung von z.B. m-Chlorperbenzoesäure bei einer Temperatur von 0°C bis 130°C zugetropft. Die Aufarbeitung erfolgt in üblicher Weise mit Natriumhydrogencarbonat/Natriumhydrogensulfitlösung zur Abtrennung der überschüssigen Persäure bzw. der gebildeten m-Chlorbenzoesäure. Das Produkt (Ie) befindet sich in der organischen Phase. Die Epoxidation kann auch mit anderen Mitteln wie z.B. Peressigsäure oder Perpropionsäure durchgeführt werden.

Die neuen Verbindungen der allgemeinen Formel (If)

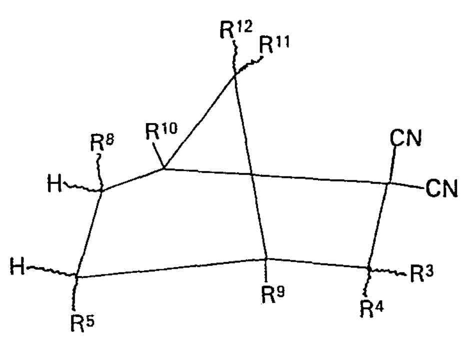

If

in welcher
$R^3$ für Chlor, Brom, Fluor, $C_1$–$C_4$-Alkyl, Trifluormethyl, Acetoxy, gegebenenfalls durch Halogen, Alkyl oder $NO_2$ substituiertes Phenyl, Cyano steht,
$R^4$ für Wasserstoff, Chlor, Brom, Fluor, $C_1$–$C_4$-Alkyl, Trifluormethyl, gegebenenfalls durch Halogen oder $NO_2$ substituiertes Phenyl steht und
$R^5$, $R^8$, $R^9$, $R^{10}$, $R^{11}$, $R^{12}$ unabhängig voneinander für Wasserstoff, Methyl, Ethyl, Halogenmethyl, Trifluormethyl, Fluor stehen,
werden hergestellt, indem man eine Verbindung der Formel (Ia) in einem Lösungsmittel katalytisch hydriert.

Als Lösungsmittel kommen z.B. in Frage: Essigester, Tetrahydrofuran, Dioxan, Cyclohexan.

Als Katalysatoren werden auf Trägern fixierte Edelmetalle, wie z.B. Platin auf Kohlenstoff oder Palladiummohr verwendet.

Die Reaktion wird bei einer Temperatur von 20°C bis 100°C und bei einem Druck von 1 bis 10 bar durchgeführt.

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Planiulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspodiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Melegethes aeneus Ptinus spp., Niptus hololeucus, Gibbium psylloides, Tribolium spp., Tenebrio molitor, Agriotes spp., Conoderus spp., Melolontha melolontha, Amphimallon solstitialris, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chrioptes spp., Sarcoptes spp., Tarsonemus spp., Bryobia praetiosa, Panonychus spp., Tetranychus spp.

Die erfindungsgemässen Stoffe weisen rodentizide Eigenschaften auf und eignen sich deshalb zur Bekämpfung von hasenartigen Tieren (Lagomorpha) und Nagetieren (Rodentia), wie Hörnchenartige (Sciuroidae), Taschenratten (Geomyoidae) und Mäuseartige (Muroidae), zu denen im wesentlichen die Haselmausartigen (Muscardinidae) und die Mäuse (Muridae) zählen.

Zu den hasenartigen Tieren gehören im wesentlichen die Leporidae, wie das Wildkaninchen (Oryctolagus cuniculus)*, zu den hörnchenartigen z.B. das Ziesel (Citellus citellus)* und das Erdhörnchen (Citellus lateralis)* und zu den Taschenratten z.B. das Mountain pocket gopher (Thomomys talpoides)*.

Zu den Haselmausartigen zählt z.B. der Siebenschläfer (Glis glis)*.

Die Mäuse umfassen im wesentlichen in der Gruppe der Langschwanzmäuse (Murinae) die Waldmäuse (Apodemus spec.); die Ratten (Rattus spec.), wie die Hausratte (Rattus rattus)* und die Wanderratte (Rattus norvegicus)*; die Hausmäuse (Mus spec.), wie Mus musculus*; in der Gruppe der Hamsterartigen (Cricetinae) den Europ. Hamster (Cricetus cricetus)* und in der Gruppe der Kurzschwanzmäuse (Microtinae) z.B. die Feldmaus (Microtus arvalis)*, die Erdmaus (Microtus agrestis)*, die grosse Wühlmaus (Arvicola terrestris)* und die Bisamratte (Ondatra zibethica).

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder

(*besonders wichtig)

Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemässen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.% Wirkstoff, vorzugsweise zwischen 0,0001 und 1 Gew.% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch als Rodentizide.

Beispiel 1

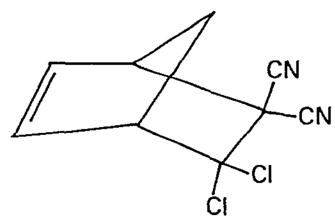

IV

100 g (1,52 Mol) monomeres Cyclopentadien werden in 300 ml Tetrahydrofuran gelöst und 220 g (1,50 Mol) 2,2-Dichlor-1-cyano-acrylnitril in 1 l Tetrahydrofuran zugetropft. Man lässt 10 Stunden bei 25°C stehen und destilliert das Lösungsmittel ab. Der Rückstand kann aus Ethanol umkristallisiert werden. Man erhält 265 g (83%) 3,3-Dichlorbycyclo[2.2.1]hept-5-en-2,2-dicarbonitril, Schmp. 161°C.

Beispiel 2

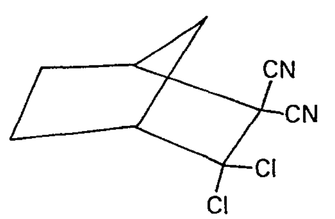

V

30 g (0,14 Mol) des in Beispiel 1 hergestellten (IV) werden in 170 ml Essigester gelöst, in einen Autoklaven gefüllt und 2 g 1%iges Platin auf Kohlenstoff zugegeben. Man erwärmt auf 40°C und presst 10 bar Wasserstoff auf. Nach ca. 2 Stunden ist die Reaktion beendet. Der Katalysator, wird abfiltriert und das Lösungsmittel verdampft. Man erhält 30 g (99%) 3,3-Dichlor-bycyclo[2.2.1]heptan-2,2-dicarbonitril, Schmp. 148°C.

Beispiel 3

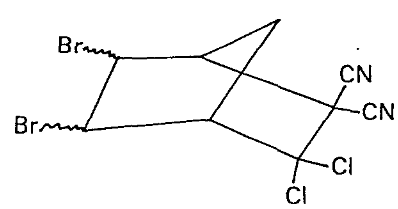

VI

100 g (0,47 Mol) des in Beispiel 1 hergestellten (IV) werden in 500 ml Trichlormethan gelöst und 75 g (0.47 Mol) Brom in 100 ml Trichlormethan zugetropft. Man erhitzt am Rückfluss, bis die Farbe des Broms verschwindet. Das Lösungsmittel wird abdestilliert und es bleiben 175 g (100%) 5,6-Dibrom-3,3-dichlor-bicyclo[2.2.1]heptan-2,2-dicarbonitril zurück, Schmp. 133°C.

Beispiel 4

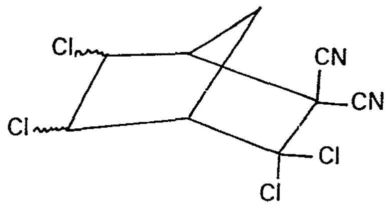

VII

100 g (0,47 Mol) des in Beispiel 1 hergestellten (IV) werden in 500 ml Chloroform gelöst und zum Sieden erhitzt. Man leitet so lange Chlorgas durch die Lösung, bis nach GC-Analyse die Ausgangsverbindung vollständig umgesetzt ist. Man erhält 130 g (98%) 3,3,5,6-Tetrachlorbicyclo[2.2.1]heptan-2,2-dicarbonitril, Schmp. 126°C (aus Ethanol).

Beispiel 5

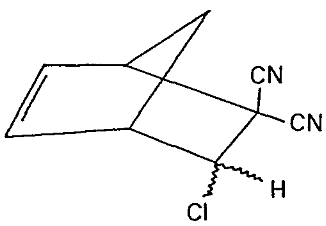

VIII

50 g (0,44 Mol) 2-Chlor-1-cyano-arylnitril und 30 g (0,45 Mol) monomeres Cyclopentadien werden in 500 ml Tetrahydrofuran gelöst und 15 Stunden bei 25°C belassen. Man zieht die flüchtigen Bestandteile im Vakuum ab und kristallisiert aus Ethanol um. Man erhält 56 g (71%) 3-Chlor-bicyclo[2.2.1]hept-5-en-2,2-dicarbonitril. Das Produkt besteht aus dem Gemisch der exo-Verbindung (VIIIa) und der endo-Verbindung VIIIb.

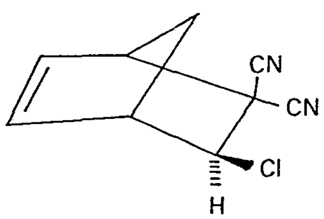

VIIIa

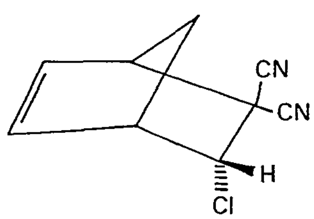

VIIIb

Durch fraktionierte Kristallisation aus Ethanol kann reines VIIIb gewonnen werden (Schmp. 131–133°C).

Beispiel 6

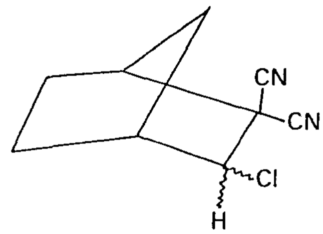

IX

30 g (0,17 Mol) des nach Beispiel 5 hergestellten (VIII) werden in 170 ml Essigester gelöst, in einen Autoklaven gefüllt, mit 3 g 1%igem Platin auf Kohlenstoff versetzt, auf 50°C erwärmt und 15 bar Wasserstoff aufgepresst. Nach 1 Stunde entspannt man den Autoklaven, filtriert den Katalysator ab und rotiert ein. Es bleiben 30 g (99%) 3-Chlor-bicyclo[2.2.1]heptan-2,2-dicarbonitril zurück, Schmp. 131°C.

Beispiel 7

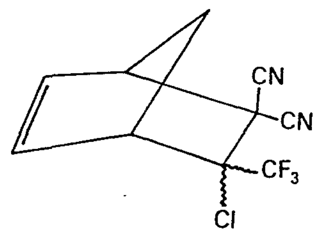

X

50 g (0,28 Mol) 2-Chlor-1-cyano-2-trifluormethyl-acrylnitril und 18,5 g (0,28 Mol) monomeres Cyclopentadien in 200 ml Dioxan werden 15 Stunden bei 25°C gehalten. Man zieht das Lösungsmittel ab und erhält 65 g (95%) 3-Chlor-3-trifluormethyl-bicyclo[2.2.1]hept-5-en-2,2-dicarbonitril, Schmp. 173°C (aus Ethanol).

Beispiel 8

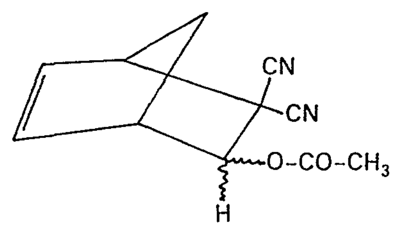

XI

20 g (0,15 Mol) 2-Acetoxy-1-cyano-acrylnitril und 15 g (0,23 Mol) monomeres Cyclopentadien werden in 150 ml Tetrahydrofuran 16 Stunden bei 25°C gehalten. Man zieht alle flüchtigen Anteile in Vakuum ab und erhält 26 g (87%) 3-Acetoxy-bicyclo[2.2.1]hept-5-en-2,2-dicarbonitril, Schmp. 133°C (aus Tetrahydrofuran).

Beispiel 9

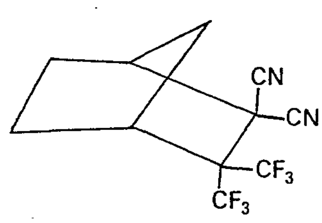

XIII

10 g (0,036 Mol) 3,3-bis-Trifluormethyl-bicyclo[2.2.1]hept-5-en-2,2-dicarbonitril [W. J. Middleton, J. Org. Chem. 30, 1402 (1965)] werden in 150 ml Essigester gelöst und in einen Autoklaven gefüllt. Man fügt 2 g 5%iges Pt/C zu, erwärmt auf 27°C und presst 7 bar Wasserstoff auf. Nach 15 Minuten entspannt man den Autoklaven, filtriert den Katalysator ab und rotiert ein. Es bleiben 10 g (99%) 3,3-bis-Trifluormethyl-bicyclo[2.2.1]heptan-2,2-dicarbonitril zurück, Schmp. 178°C.

Beispiel 10

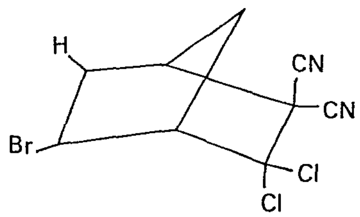

XIVa

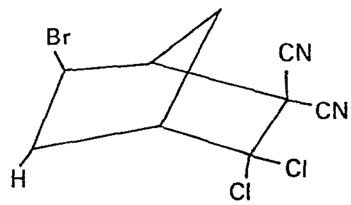

XIVb

50 g (0,13 Mol) des in Beispiel 3 hergestellten (VI) werden in 300 ml Toluol gelöst und 30,4 g (0,20 Mol) Diazabicycloundecen (DBU) in 200 ml Toluol zugetropft. Man erhitzt 1 Stunde zum Sieden, kühlt ab und schüttelt mit 1 N Salzsäure aus. Man trocknet die org. Phase mit Magnesiumsulfat und rotiert ein. Es bleiben 30,5 g (78%) eines Gemisches aus 5-Brom-3,3-dichlor-bicyclo[2.2.1]hept-5-en-2,2-dicarbonitril (XIVa) und 6-Brom-3,3-dichlor-bicyclo[2.2.1]hept-5-en-2,2-dicarbonitril (XIVb) zurück, Schmelzbereich 105–107°C.

Beispiel 11

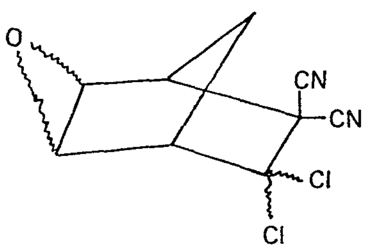

XVI

21 g (0,1 Mol) des im Beispiel 1 hergestellten (IV) werden in 100 ml Chlorbenzol gelöst und 20 g (0,12 Mol) m-Chlorperbenzoesäure in 200 ml Chlorbenzol zugetropft. Man erhitzt 4 Stunden zum Rückfluss und arbeitet in üblicher Weise mit Natriumhydrogensulfidlösung zur Zerstörung des Persäureüberschusses und Natriumhydrogencarbonatlösung zur Abtrennung der m-Chlorbenzoesäure auf. Aus der organischen Phase wurden 17 g (75%) 3,3-Dichlor-5,6-epoxy-bicyclo[2.2.1]heptan-2,2-dicarbonitril isoliert, Schmp. 229°C.

In den folgenden Verwendungsbeispielen werden Formeln der Verbindungen angegeben, ohne dass damit Aussagen über die sterischen Verhältnisse gemacht werden.

Beispiel A

Lt$_{100}$-Test für Dipteren

Testtiere: Musca domestica

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro m² Filterpapier verschieden hoch. Anschliessend gibt man etwa 25 Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Testtiere, Wirkstoffe, Wirkstoffkonzentrationen und Zeiten, bei denen eine 100%ige knock down-Wirkung vorliegt, gehen aus der nachfolgenden Tabelle hervor:

Tabelle

LT$_{100}$-Test für Dipteren (Musca domestica)

| Wirkstoffe | Wirkstoffkonzentration der Lösung in % | LT$_{100}$ |
|---|---|---|
| Bekannt: | | |
| Toxaphen | 0,2 | $4^h$ = 0% |
| 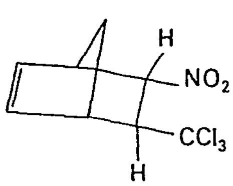 | 0,02 | $6^h$ = 90% |

Tabelle (Fortsetzung)
$LT_{100}$-Test für Dipteren (Musca domestica)

| Wirkstoffe | | Wirkstoffkonzentration der Lösung in % | $LT_{100}$ |
|---|---|---|---|
| Erfindungsgemäss: | | | |
| 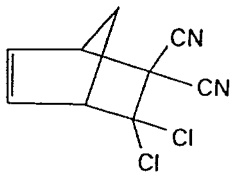 | | 0,02 | 90′ |
| 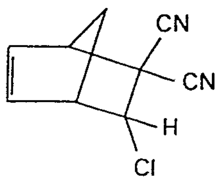 | | 0,02 | 110′ |
| 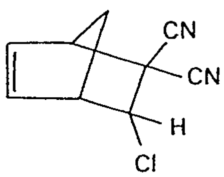 | 10% exo<br>90% endo | 0,02 | 85′ |
| 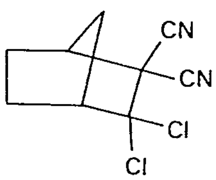 | | 0,02 | 110′ |
| 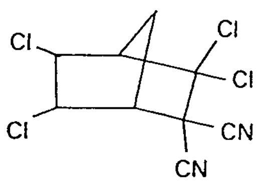 | | 0,002 | 150′ |
| 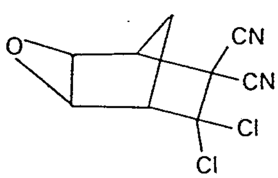 | | 0,02 | 55′ |
| 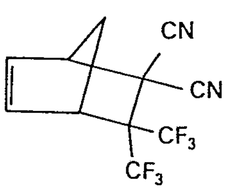 | | 0,0002 | 75′ |
| 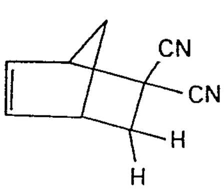 | | 0,02 | 90′ |

Tabelle (Fortsetzung)
$LT_{100}$-Test für Dipteren (Musca domestica)

| Wirkstoffe | Wirkstoffkonzentration der Lösung in % | $LT_{100}$ |
|---|---|---|
| 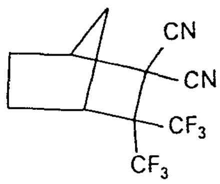 | 0,02 | 65′ |
| 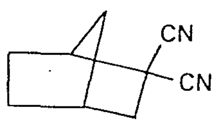 | 0,02 | 90′ |

Beispiel B

$LT_{100}$-Test für Dipteren

Testtiere: Aëdes aegypti

Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro m² Filterpapier verschieden hoch. Anschliessend gibt man etwa 25 Testtiere in die Petrischale und bedeckt sie mit einem Glasdeckel.

Der Zustand der Testtiere wird laufend kontrolliert. Es wird diejenige Zeit ermittelt, welche für einen 100%igen knock down-Effekt notwendig ist.

Testtiere, Wirkstoffe, Wirkstoffkonzentrationen und Zeiten, bei denen eine 100%ige knock down-Wirkung vorliegt, gehen aus der nachfolgenden Tabelle hervor:

Tabelle
$LT_{100}$-Test für Dipteren (Aëdes aegypti)

| Wirkstoffe | Wirkstoffkonzentration der Lösung in % | $LT_{100}$ |
|---|---|---|
| Bekannt: | | |
| 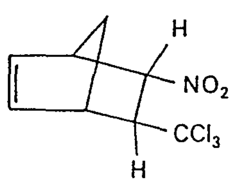 | 0,02 | 180′ |
| Erfindungsgemäss: | | |
| 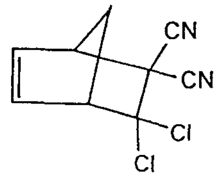 | 0,002 | 60′ |
| 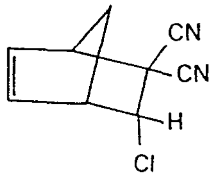 endo/exo-Gemisch | 0,002 | 120′ |

Tabelle (Fortsetzung)
$LT_{100}$-Test für Dipteren (Aëdes aegypti)

| Wirkstoffe | | Wirkstoffkonzentration der Lösung in % | $LT_{100}$ |
|---|---|---|---|
| 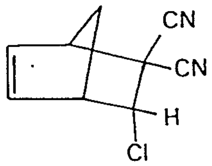 | 90% endo + 10% exo | 0,002 | 120′ |
| 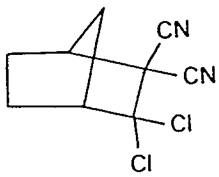 | | 0,002 | 120′ |
| 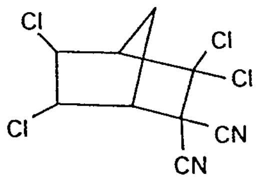 | | 0,002 | 60′ |
| 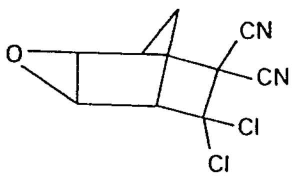 | | 0,002 | 60′ |
| 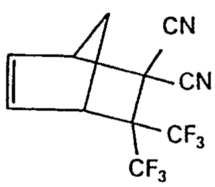 | | 0,002 | 60′ |
| 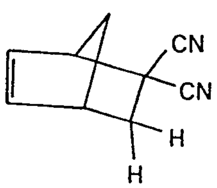 | | 0,02 | 60′ |
| 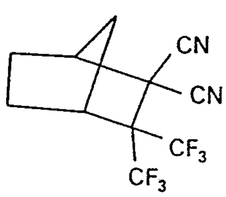 | | 0,02 | 60′ |
| 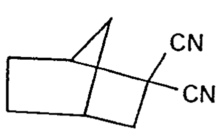 | | 0,02 | 60′ |

Tabelle (Fortsetzung)
$LT_{100}$-Test für Dipteren (Aëdes aegypti)

| Wirkstoffe | Wirkstoffkonzentration der Lösung in % | $LT_{100}$ |
|---|---|---|
| 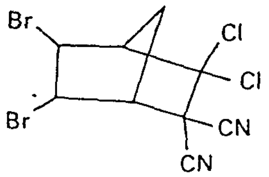 | 0,02 | 60′ |
| 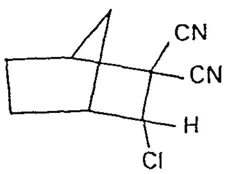 | 0,02 | 60′ |
| 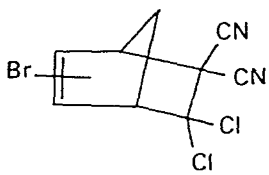 | 0,02 | 60′ |

**Beispiel C**

Drosophila-Test

Lösungsmittel: 3 Gewichtsteile Dimethylformamid

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

1 $cm^3$ der Wirkstoffzubereitung wird auf eine Filterpapierscheibe (7 cm Durchmesser) aufpipettiert. Man legt diese nass auf die Öffnung eines Glasgefässes, in dem sich 50 Taufliegen (Drosophila melanogaster) befinden und bedeckt es mit einer Glasplatte.

Nach der gewünschten Zeit bestimmt man die Abtötung in %. Dabei bedeutet 100%, dass alle Fliegen abgetötet wurden; 0% bedeutet, dass keine Fliegen abgetötet wurden.

Bei diesem Test zeigen z.B. die folgenden Verbindungen der Herstellungsbeispiele überlegene Wirksamkeit gegenüber dem Stand der Technik:

Tabelle
(pflanzenschädigende Insekten)
Drosophila-Test

| Wirkstoffe | Wirkstoffkonzentration in % | Abtötungsgrad in % nach 1 d |
|---|---|---|
| Toxaphen (bekannt) | 0,1 | 0 |
| 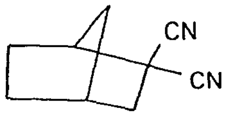 | 0,1 | 100 |
| 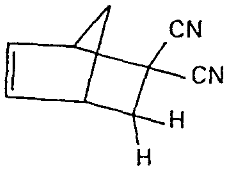 | 0,1 | 100 |

Tabelle (Fortsetzung)
(pflanzenschädigende Insekten)
Drosophila-Test

| Wirkstoffe | Wirkstoffkonzentration in % | Abtötungsgrad in % nach 1 d |
|---|---|---|
| 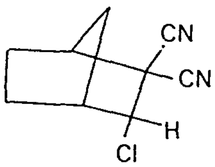 exo/endo | 0,1 | 100 |
| 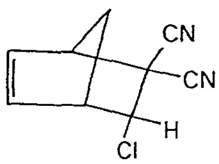 exo/endo | 0,1 | 100 |
| 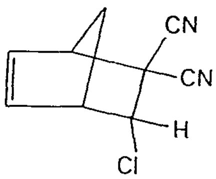 90˙/.endo/10˙/.exo | 0,1 | 100 |
| 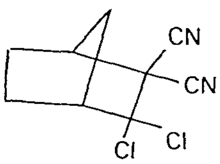 | 0,1 | 100 |
| 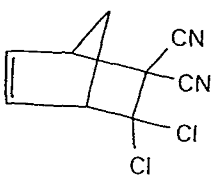 | 0,1 | 100 |
| 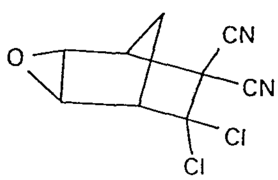 | 0,1 | 100 |
| 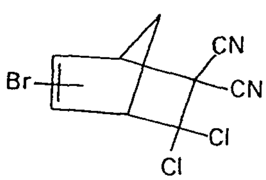 | 0,1 | 100 |
| 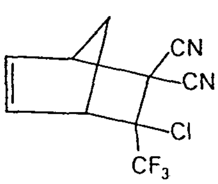 | 0,1 | 100 |

Tabelle (Fortsetzung)
(pflanzenschädigende Insekten)
Drosophila-Test

| Wirkstoffe | Wirkstoffkonzentration in % | Abtötungsgrad in % nach 1 d |
|---|---|---|
| 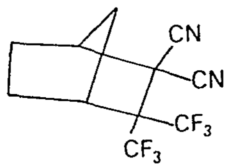 | 0,1 | 100 |
| 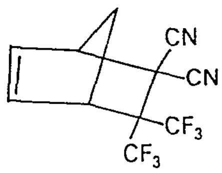 | 0,1 | 100 |
| 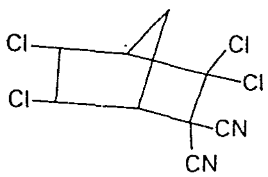 | 0,1 | 100 |
| 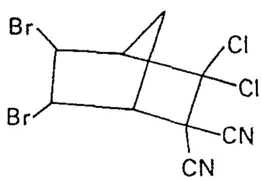 | 0,1 | 100 |

Beispiel D
Doralis-Test (systemische Wirkung)

Lösungsmittel: 3 Gewichtsteile Dimethylformamid
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so dass die Wirkstoffzubereitung in den Boden eindringt, ohne den Spross zu benetzen. Der Wirkstoff wird von den Wurzeln aufgenommen und in den Spross weitergeleitet.

Nach der gewünschten Zeit wird die Abtötung in % bestimmt. Dabei bedeutet 100%, dass alle Blattläuse abgetötet wurden; 0% bedeutet, dass keine Blattläuse abgetötet wurden.

Bei diesem Test zeigen z.B. die folgenden Verbindungen der Herstellungsbeispiele überlegene Wirksamkeit gegenüber dem Stand der Technik:

Tabelle
(pflanzenschädigende Insekten)
Doralis fabae-Test (syst. Wirkung)

| Wirkstoffe | Wirkstoffkonzentration in % | Abtötungsgrad in % nach 4 d |
|---|---|---|
| Toxaphen (bekannt) | 0,1 | 0 |
| 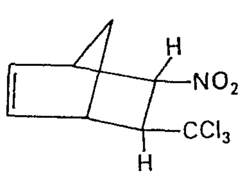 (bekannt) | 0,1 | 0 |

Tabelle (Fortsetzung)
(pflanzenschädigende Insekten)
Doralis fabae-Test (syst. Wirkung)

| Wirkstoffe | Wirkstoffkonzentration in % | Abtötungsgrad in % nach 4 d |
|---|---|---|
| 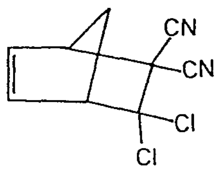 | 0,1 | 100 |
| 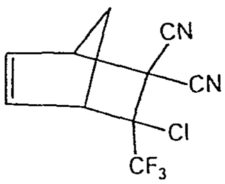 | 0,1 | 100 |
| 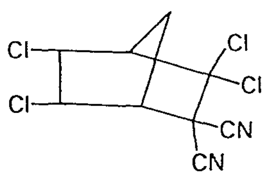 | 0,1 | 85 |

Beispiel E
Tetranychus-Test (resistent)

Lösungsmittel: 3 Gewichtsteile Dimethylformamid
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Spinnmilben abgetötet wurden; 0% bedeutet, dass keine Spinnmilben abgetötet wurden.

Bei diesem Test zeigen z.B. die folgenden Verbindungen der Herstellungsbeispiele überlegene Wirksamkeit gegenüber dem Stand der Technik:

Tabelle
(pflanzenschädigende Milben)
Tetranychus-Test

| Wirkstoffe | Wirkstoffkonzentration in % | Abtötungsgrad in % nach 2 d |
|---|---|---|
| Toxaphen<br>(bekannt) | 0,1<br>0,01 | 98<br>0 |
| 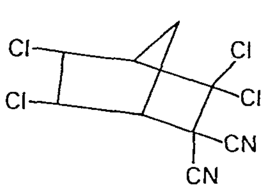 | 0,1<br>0,01 | 100<br>35 |

Beispiel F

Grenzkonzentrations-Test/Bodeninsekten

Testinsekt: Phorbia antiqua-Maden im Boden
Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und lässt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Wirkstoffe, Aufwandmengen und Resultate gehen aus der nachfolgenden Tabelle hervor:

Tabelle
Bodeninsektizide
Phorbia antiqua-Maden im Boden

| Wirkstoff (Konstitution) | Abtötungsgrad in % bei Wirkstoffkonzentration in ppm 10 |
|---|---|
| Toxaphen (bekannt) | 0% |
| 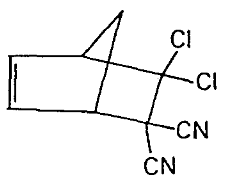 | 100% |
| 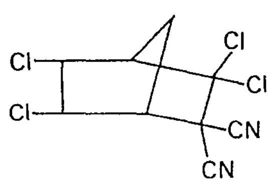 | 100% |
| 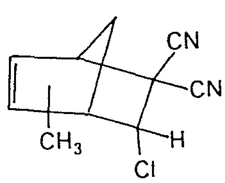 | 100% |
| 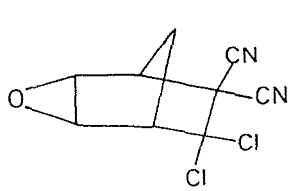 | 100% |
| 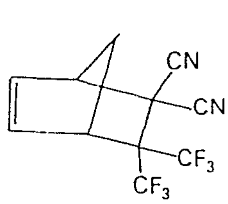 | 100% |

Tabelle (Fortsetzung)
Bodeninsektizide
Phorbia antiqua-Maden im Boden

| Wirkstoff (Konstitution) | Abtötungsgrad in % bei Wirkstoffkonzentration in ppm 10 |
|---|---|
| 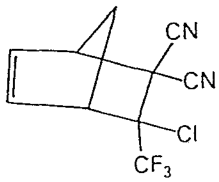 | 100% |

Beispiel G

Grenzkonzentrations-Test/Bodeninsekten

Testinsekt: Tenebrio molitor-Larven im Boden
Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und lässt diese bei Raumtemperaturen stehen.

Nach 24 Stunden werden die Testtiere in den behandelten Boden gegeben und nach weiteren 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Wirkstoffe, Aufwandmengen und Resultate gehen aus der nachfolgenden Tabelle hervor:

Tabelle
Bodeninsektizide
Tenebrio molitor-Larven im Boden

| Wirkstoff (Konstitution) | Abtötungsgrad in % bei Wirkstoffkonzentration in ppm 10 |
|---|---|
| Toxaphen (bekannt) | 0% |
| 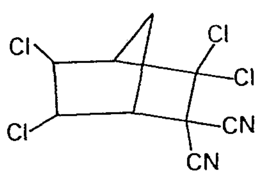 | 100% |
| 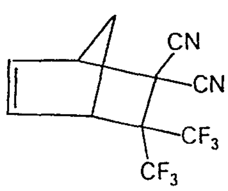 | 100% |
| 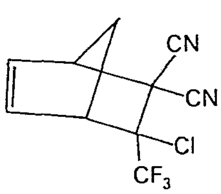 | 100% |

**Patentansprüche**

1. Verwendung von substituierten Bicyclen der Formel (I) zur Bekämpfung von Schädlingen

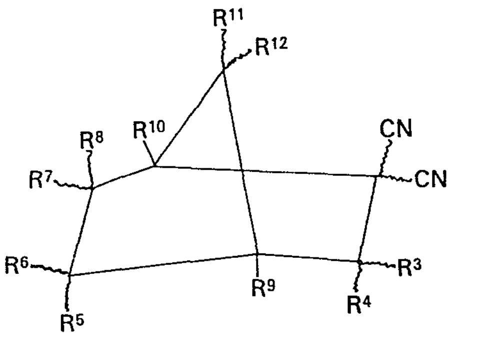

I

in welcher
$R^3$ für Wasserstoff, $C_1$–$C_4$-Alkyl, gegebenenfalls durch Halogen, Alkyl oder $NO_2$ substituiertes Phenyl, Halogen, $C_{1-4}$-Halogenalkyl, Acetoxy, $C_1$–$C_4$-Alkoxycarbonyl, Aralkoxycarbonyl, Phenyloxycarbonyl, Cyano, Nitro, Hydroxy, $C_1$–$C_4$-Alkoxy, Phenyloxy,
$R^4$ für Wasserstoff, $C_1$–$C_4$-Alkyl, gegebenenfalls durch Halogen oder $NO_2$ substituiertes Phenyl, Halogen, $C_{1-4}$-Halogenalkyl, $C_{1-4}$-Alkoxycarbonyl oder $R^3$ und $R^4$ zusammen für Dihalogenmethylen,
$R^5$, $R^6$, $R^7$, $R^8$ unabhängig voneinander für Wasserstoff, $C_1$–$C_4$-Alkyl, Phenyl, Halogen, $C_{1-4}$-Halogenalkyl, $C_1$–$C_4$-Alkylthio, Phenylthio, Phenoxy, $C_1$–$C_4$-Alkoxy und darüber hinaus $R^5$ und $R^6$ zusammen für eine Doppelbindung oder Sauerstoff oder
$R^6$ und $R^7$ zusammen für eine Doppelbindung oder für Sauerstoff,
$R^9$ und $R^{10}$ unabhängig voneinander für Wasserstoff, $C_1$–$C_4$-Alkyl, $C_{1-4}$-Halogenalkyl, Fluor,
$R^{11}$ und $R^{12}$ unabhängig voneinander für Wasserstoff, $C_1$–$C_4$-Alkyl, $C_{1-4}$-Halogenalkyl, Halogen oder $R^{11}$ und $R^{12}$ zusammen für Ethyliden, Isopropyliden stehen.

2. Verfahren zur Bekämpfung von Schädlingen, dadurch gekennzeichnet, dass man substituierte Bicyclen der Formel (I) gemäss Anspruch 1 auf Schädlinge und/oder ihren Lebensraum einwirken lässt.

3. Verfahren zur Herstellung von Schädlingsbekämpfungsmitteln, dadurch gekennzeichnet, dass man substituierte Bicyclen der Formel (I) gemäss Anspruch 1 mit Streckmitteln und/oder oberflächenaktiven Mitteln vermischt.

4. Schädlingsbekämpfungsmittel, gekennzeichnet durch einen Gehalt an mindestens einem substituierten Bicyclus der allgemeinen Formel I gemäss Anspruch 1 mit Ausnahme von 3,3-bis-Trifluormethyl-bicyclo[2.2.1] hept-5-en-2,2-dicarbonitril.

5. Schädlingsbekämpfungsmittel gemäss Anspruch 4, dadurch gekennzeichnet, dass in Formel I
$R^3$ für Wasserstoff, Methyl, Ethyl, Brom, Chlor, Fluor, Cyano, Trifluormethyl, Trichlormethyl, Acetoxy, Hydroxy, Dichlorfluormethyl, Chlordifluormethyl, Bromdichlormethyl, Phenyl, o-Chlorphenyl, p-Nitrophenyl, Methoxycarbonyl, Ethoxycarbonyl, Pentafluorbenzyloxycarbonyl, Phenoxycarbonyl steht,
$R^4$ für Wasserstoff, Methyl, Ethyl, Brom, Chlor, Fluor, Trifluormethyl, Trichlormethyl, Dichlorfluormethyl, Chlordifluormethyl, Bromdichlormethyl, Phenyl, o-Chlorphenyl, p-Nitrophenyl, Methoxycarbonyl, Ethoxycarbonyl steht,
$R^3$ und $R^4$ zusammen für Dichlormethylen stehen,
$R^5$, $R^6$, $R^7$, $R^8$ unabhängig voneinander für Wasserstoff, Methyl, Brom, Chlor, Fluor, Methylthio, Phenylthio stehen,
$R^6$ und $R^7$ zusammen für eine Doppelbindung oder Sauerstoff stehen,
$R^9$ und $R^{10}$ unabhängig voneinander für Wasserstoff, Methyl, Trifluormethyl, Fluor stehen,
$R^{11}$ und $R^{12}$ unabhängig voneinander für Wasserstoff, Methyl, Fluor, Brom, Chlor, 1-Bromethyl, 1-Chlorethyl, 1-Brom-1-Methylethyl, 1-Chlor-1-Methylethyl stehen,
$R^{11}$ und $R^{12}$ zusammen für Ethyliden, Isopropyliden stehen.

**Claims**

1. Use of substituted bicyclic compounds of the formula (I) for combating pests

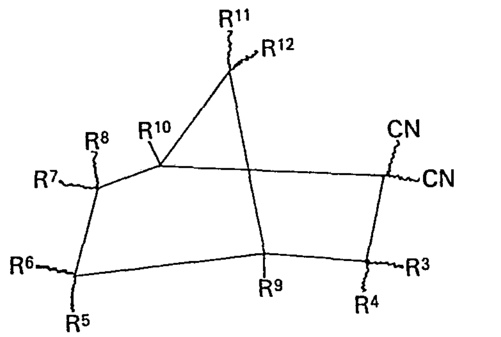

I

in which
$R^3$ represents hydrogen, $C_1$–$C_4$-alkyl, optionally halogen-, alkyl- or $NO_2$-substituted phenyl, halogen, $C_{1-4}$-halogenoalkyl, acetoxy, $C_1$–$C_4$-alkoxycarbonyl, aralkoxycarbonyl, phenyloxycarbonyl, cyano, nitro, hydroxyl, $C_1$–$C_4$-alkoxy or phenyloxy,
$R^4$ represents hydrogen, $C_1$–$C_4$-alkyl, optionally halogen- or $NO_2$-substituted phenyl, halogen, $C_{1-4}$-halogenoalkyl, $C_{1-4}$-alkoxycarbonyl or $R^3$ and $R^4$ together represent dihalogenomethylene,
$R^5$, $R^6$, $R^7$ and $R^8$ independently of one another represent hydrogen, $C_1$–$C_4$-alkyl, phenyl, halogen, $C_{1-4}$-halogenalkyl, $C_1$–$C_4$-alkylthio, phenylthio, phenoxy, $C_1$–$C_4$-alkoxy, and also $R^5$ and $R^6$ together represent a double bond or oxygen or $R^6$ and $R^7$ together represent a double bond or oxygen,
$R^9$ and $R^{10}$ independently of one another represent hydrogen, $C_1$–$C_4$-alkyl, $C_{1-4}$-halogenoalkyl or fluorine,
$R^{11}$ and $R^{12}$ independently of one another represent hydrogen, $C_1$–$C_4$-alkyl, $C_{1-4}$-halogenoalkyl, halogen or $R^{11}$ and $R^{12}$ together represent ethylidene or isopropylidene.

2. Method of combating pests, characterised

in that substituted bicyclic compounds of the formula (I) according to Claim 1 are allowed to act on pests and/or their environment.

3. Process for the preparation of agents for combating pests, characterised in that substituted bicyclic compounds of the formula (I) according to Claim 1 are mixed with extenders and/or surface-active agents.

4. Agents for combating pests, characterised in that they contain at least one substituted bicyclic compound of the general formula I according to Claim 1, with the exception of 3,3-bis-trifluoromethyl-bicyclo[2.2.1]hept-5-ene-2,2-dicarbonitrile.

5. Agents for combating pests according to Claim 4, characterised in that in the formula I
$R^3$ represents hydrogen, methyl, ethyl, bromine, chlorine, fluorine, cyano, trifluoromethyl, trichloromethyl, acetoxy, hydroxyl, dichlorofluoromethyl, chlordifluoromethyl, bromodichloromethyl, phenyl, o-chlorophenyl, p-nitrophenyl, methoxycarbonyl, ethoxycarbonyl, pentafluorobenzyloxycarbonyl or phenoxycarbonyl,
$R^4$ represents hydrogen, methyl, ethyl, bromine, chlorine, fluorine, trifluoromethyl, trichloromethyl, dichlorofluoromethyl, chlordifluoromethyl, bromodichloromethyl, phenyl, o-chlorophenyl, p-nitrophenyl, methoxycarbonyl or ethoxycarbonyl,
$R^3$ and $R^4$ together represent dichloromethylene,
$R^5$, $R^6$, $R^7$ and $R^8$ independently of one another represent hydrogen, methyl, bromine, chlorine, fluorine, methylthio or phenylthio,
$R^6$ and $R^7$ together represent a double bond or oxygen,
$R^9$ and $R^{10}$ independently of one another represent hydrogen, methyl, trifluoromethyl or fluorine,
$R^{11}$ and $R^{12}$ independently of one another represent hydrogen, methyl, fluorine, bromine, chlorine, 1-bromoethyl, 1-chloroethyl, 1-bromomethylethyl or 1-chloro-1-methylethyl, and
$R^{11}$ and $R^{12}$ together represent ethylidine or isopropylidene.

**Revendications**

1. Utilisatio de composés bicycliques substitués de formule (I) pour combattre des parasites

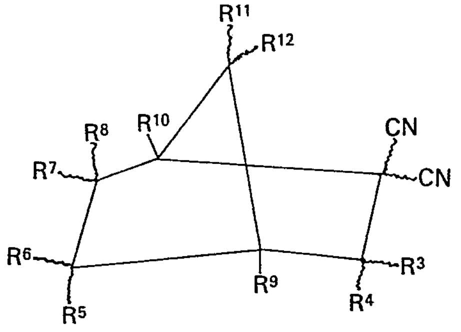

I

formule dans laquelle
$R^3$ représente l'hydrogène, un groupe alkyle en $C_1$ à $C_4$, un groupe phényle éventuellement substitué par un radical halogéno, alkyle ou $NO_2$, un halogène, un groupe halogénalkyle en $C_1$ à $C_4$, (alkoxy en $C_1$ à $C_4$)-carbonyle, aralkoxycarbonyle, phényloxycarbonyle, cyano, Nitro, hydroxy, alkoxy en $C_1$ à $C_4$, phényloxy,
$R^4$ désigne l'hydrogène, un groupe alkyle en $C_1$ à $C_4$, phényle éventuellement substitué par un radical halogéno ou $NO_2$, un halogène, un grope halogénalkyle en $C_1$ à $C_4$, (alkoxy en $C_1$ à $C_4$)carbonyle ou bien $R^3$ et $R^4$ forment ensemble en groupe dihalogénométhylène,
$R^5$, $R^6$, $R^7$, $R^8$ représentent indépendamment les uns des autres l'hydrogène ou un groupe alkyle en $C_1$ à $C_4$, un groupe phényle, un halogène, un groupe halogénalkyle en $C_1$ à $C_4$, alkylthio en $C_1$–$C_4$, phénylthio, phénoxy, alkoxy en $C_1$ à $C_4$, etc., $R^5$ et $R^6$ forment ensemble une double liaison ou un atome d'oxygène, ou bien
$R^6$ et $R^7$ forment ensemble une double liaison ou un atome d'oxygène,
$R^9$ et $R^{10}$ représentent indépendamment l'un de l'autre l'hydrogène, un groupe alkyle en $C_1$ à $C_4$, halogénalkyle en $C_1$ à $C_4$, du fluor,
$R^{11}$ et $R^{12}$ représentent indépendamment l'un de l'autre de l'hydrogène, un groupe alkyle en $C_1$ à $C_4$, halogénalkyle en $C_1$ à $C_4$, un halogène, ou bien $R^{11}$ et $R^{12}$ forment ensemble un groupe éthylidène, isopropylidène.

2. Procédé pour combattre des parasites, caractérisé en ce qu'on fait agir des composés bicycliques substitués de formule (I) suivant la revendication 1 sur des parasites et/ou sur leur habitat.

3. Procédé de préparation de compositions pesticides, caractérisé en ce qu'on mélange des composés bicycliques substitués de formule (I) suivant la revendication 1 avec des diluants et/ou des agents tensio-actifs.

4. Compositions pesticides, caractérisées par une teneur en au moins un composé bicyclique substitué de formule générale I suivant la revendication 1, à l'exception du 3,3-bis-trifluorométhylbicyclo[2.2.1]hept-5-ène-2,2-dicarbonitrile.

5. Compositions pesticides suivant la revendication 4,caractérisées en ce que,dans la formule I,
$R^3$ représente l'hydrogène, un groupe méthyle, éthyle, le brome, le chlore, le fluor, un groupe cyano, trifluorométhyle, trichlorométhyle, acétoxy, hydroxy, dichlorofluorométhyle, chlorodifluorométhyle, bromodichlorométhyle, phényle, o-chlorophényle, p-nitrophényle, méthoxycarbonyle, éthoxycarbonyle, pentafluorobenzyloxycarbonyle, phénoxycarbonyle,
$R^4$ représente l'hydrogène, un groupe méthyle, éthyle, du brome, du chlore, du fluor, un groupe trifluorométhyle, trichlorométhyle, dichlorofluorométhyle, chlorodifluorométhyle, bromodichlorométhyle, phényle, o-chlorophényle, p-nitrophényle, méthoxycarbonyle, éthoxycarbonyle,
$R^3$ et $R^4$ forment conjointement un groupe dichlorométhylène,
$R^5$, $R^6$, $R^7$, $R^8$ représentent indépendamment les uns des autres l'hydrogène, un groupe méthyle, le brome, le chlore, le fluor, un groupe méthylthio, phénylthio,

$R^6$ et $R^7$ forment conjointement une double liaison ou un atome d'oxygène,
$R^9$ et $R^{10}$ représentent indépendamment l'un de l'autre de l'hydrogène, un groupe méthyle, trifluorométhyle, du fluor,
$R^{11}$ et $R^{12}$ représentent indépendamment l'un de l'autre l'hydrogène, un groupe méthyle, du fluor, du brome, du chlore, un groupe 1-brométhyle, 1-chloréthyle, 1-bromo-1-méthyléthyle, 1-chloro-1-méthyléthyle,
$R^{11}$ et $R^{12}$ forment ensemble un groupe éthylidène, isopropylidène.